(19) Europäisches Patentamt
European Patent Office
Office européen des brevets

(11) Veröffentlichungsnummer : **0 086 980**
**B1**

(12) # EUROPÄISCHE PATENTSCHRIFT

(45) Veröffentlichungstag der Patentschrift : **02.01.86**

(21) Anmeldenummer : **83100676.2**

(22) Anmeldetag : **26.01.83**

(51) Int. Cl.⁴ : **C 07 D401/12**, C 07 D243/38, C 07 D451/04, C 07 D451/12, A 61 K 31/55

(54) **Substituierte Dibenzodiazepinone, Verfahren zu ihrer Herstellung und diese Verbindungen enthaltende Arzneimittel.**

(30) Priorität : **06.02.82 DE 3204158**

(43) Veröffentlichungstag der Anmeldung : **31.08.83 Patentblatt 83/35**

(45) Bekanntmachung des Hinweises auf die Patenterteilung : **02.01.86 Patentblatt 86/01**

(84) Benannte Vertragsstaaten : **AT BE CH DE FR GB IT LI LU NL SE**

(56) Entgegenhaltungen :
**EP-A- 0 044 989**
**DE-A- 1 795 176**
**DE-A- 1 936 670**
**Die Akte enthält technische Angaben, die nach dem Eingang der Anmeldung eingereicht wurden und die nicht in dieser Patentschrift enthalten sind.**

(73) Patentinhaber : **Dr. Karl Thomae GmbH**
**Postfach 1755**
***D-7950 Biberach (Riss) (DE)***

(72) Erfinder : **Engel, Wolfhard, Dr. Dipl.-Chem.**
**Mozartstrasse 13**
**D-7950 Biberach 1 (DE)**
Erfinder : **Trummlitz, Günter, Dr. Dipl.-Chem.**
**Buchenweg 27**
**D-7951 Warthausen (DE)**
Erfinder : **Schmidt, Günther, Dr. Dipl.-Chem.**
**Johann-Seb.-Bach-Strasse 27**
***D-7950 Biberach (DE)***
Erfinder : **Eberlein, Wolfgang, Dr. Dipl.-Chem.**
**Obere Au 6**
**D-7950 Biberach 1 (DE)**
Erfinder : **Hammer, Rudolf, Dr.**
**Via Fabio Filzi 33**
**Milano (IT)**
Erfinder : **Del Soldato, Piero, Dr.**
**Via E. Toti 22**
**Monza (IT)**

**EP 0 086 980 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue substituierte Dibenzodiazepinone, Verfahren zu ihrer Herstellung und diese Verbindungen enthaltende Arzneimittel.

In der deutschen Offenlegungsschrift DE-OS 1 795 176 werden bestimmte Dibenzodiazepinone mit einer ulcushemmenden und sekretionshemmenden Wirkung beschrieben. Aus der US-Patentschrift US-PS 3 953 430 sind substituierte Dibenzodiazepine mit antidepressiver und analgetischer Wirkung bekannt.

Es wurden nun Dibenzodiazepinone mit neuartigen Aminoacylresten gefunden, die gegenüber den oben erwähnten Verbindungen interessante und überlegene pharmakologische Wirkungen aufweisen.

Gegenstand der Erfindung sind :

a) substituierte Dibenzodiazepinone der allgemeinen Formel I

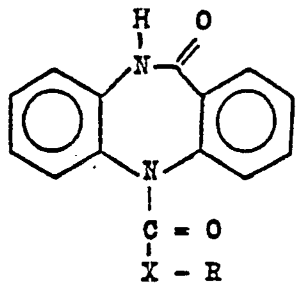

(I)

worin

X ein Sauerstoffatom, eine —NH— oder —$NCH_3$-Gruppe und

R einen — gegebenenfalls durch eine weitere Methylgruppe substituierten — 1-Methyl-4-piperidinyl-, 4-Methyl-1-piperazinyl- oder einen 3α- oder 3β-Tropanyl-Rest bedeuten,

sowie ihre Säureadditionssalze.

Die Verbindungen der allgemeinen Formel I können nach Umsetzung mit anorganischen oder organischen Säuren auch in Form ihrer physiologisch verträglichen Salze vorliegen. Als Säuren haben sich beispielsweise Salzsäure, Bromwasserstoffsäure, Schwefelsäure, Phosphorsäure, Weinsäure, Fumarsäure, Citronensäure, Maleinsäure, Bernsteinsäure, Gluconsäure, Apfelsäure, p-Toluolsulfonsäure, Methansulfonsäure oder Amidosulfonsäure als geeignet erwiesen.

Zur Erläuterung des Erfindungsgegenstandes seien als Beispiele folgende Verbindungen genannt :

5,10-Dihydro-5-[[(1-methyl-4-piperidinyl)oxy]carbonyl]-11H-dibenzo[b,e][1,4]diazepin-11-on

cis-5,10-Dihydro-5-[[(1,2-dimethyl-4-piperidinyl)oxy]carbonyl]-11H-dibenzo[b,e][1,4]diazepin-11-on

trans-5,10-Dihydro-5-[[(1,2-dimethyl-4-piperidinyl)oxy]carbonyl]-11H-dibenzo[b,e][1,4]diazepin-11-on

cis-5,10-Dihydro-5-[[(1,3-dimethyl-4-piperidinyl)oxy]carbonyl]-11H-dibenzo[b,e][1,4]diazepin-11-on

trans-5,10-Dihydro-5-[[(1,3-dimethyl-4-piperidinyl)oxy]carbonyl]-11H-dibenzo[b,e][1,4]diazepin-11-on

5,10-Dihydro-5-[[(1-methyl-4-piperidinyl)amino]carbonyl]-11H-dibenzo[b,e][1,4]diazepin-11-on

cis-5,10-Dihydro-5-[[(1,2-dimethyl-4-piperidinyl)amino]carbonyl]-11H-dibenzo[b,e][1,4]diazepin-11-on

trans-5,10-Dihydro-5-[[(1,2-dimethyl-4-piperidinyl)amino]-carbonyl]-11H-dibenzo[b,e][1,4]diazepin-11-on

cis-5,10-Dihydro-5-[[(1,3-dimethyl-4-piperidinyl)amino]carbonyl]-11H-dibenzo[b,e][1,4]diazepin-11-on

trans-5,10-Dihydro-5-[[(1,3-dimethyl-4-piperidinyl)amino]-carbonyl]-11H-dibenzo[b,e][1,4]diazepin-11-on

5,10-Dihydro-5-[[(4-methyl-1-piperazinyl)-amino]carbonyl]-11H-dibenzo[b,e][1,4]diazepin-11-on

5,10-Dihydro-5-[[(3,4-dimethyl-1-piperazinyl)-amino]carbonyl]-11H-dibenzo[b,e][1,4]diazepin-11-on

5,10-Dihydro-5-[[(2,4-dimethyl-1-piperazinyl)amino]carbonyl]-11H-dibenzo[b,e][1,4]diazepin-11-on

5,10-Dihydro-5-[[(4-methyl-1-piperazinyl)oxy]carbonyl]-11H-dibenzo[b,e][1,4]diazepin-11-on

5,10-Dihydro-5-[[(3,4-dimethyl-1-piperazinyl)oxy]carbonyl]-11H-dibenzo[b,e][1,4]diazepin-11-on

5,10-Dihydro-5-[[(2,4-dimethyl-1-piperazinyl)oxy]carbonyl]-11H-dibenzo[b,e][1,4]diazepin-11-on

endo-5,10-Dihydro-5-[[(8-methyl-8-azabicyclo[3,2,1]oct-3-yl)-oxy]-carbonyl]-11H-dibenzo-[b,e][1,4]diazepin-11-on

exo-5,10-Dihydro-5-[[(8-methyl-8-azabicyclo[3,2,1]oct-3yl)-oxy]carbonyl]-11H-dibenzo[b,e][1,4]diazepin-11-on

endo-5,10-Dihydro-5-[[(8-methyl-8-azabicyclo[3,2,1]oct-3-yl)-oxy]carbonyl]-11H-dibenzo[b,e][1,4]-diazepin-11-on

exo-5,10-Dihydro-5-[[(8-methyl-8-azabicyclo[3,2,1]oct-3-yl)-amino]carbonyl]-11H-dibenzo[b,e][1,4]-diazepin-11-on

5,10-Dihydro-5-[[N-methyl-N-(1-methyl-4-piperidinyl)amino]-carbonyl]-11H-dibenzo[b,e] [1,4]diaze-pin-11-on

cis-5,10-Dihydro-5-[[N-methyl-N-(1,2-dimethyl-4-piperidinyl)-amino]carbonyl]-11H-dibenzo[b,e][1,4]diazepin-11-on

trans-5,10-Dihydro-5-[[N-methyl-N-(1,2-dimethyl-4-piperidinyl)-amino]carbonyl]-11H-dibenzo[b,e][1,4]-diazepin-11-on

cis-5,10-Dihydro-5-[[N-methyl-N-(1,3-dimethyl-4-piperidinyl)-amino]carbonyl]-11H-dibenzo[b,e][1,4]-diazepin-11-on

trans-5,10-Dihydro-5-[[N-methyl-N-(1,3-dimethyl-4-piperidinyl)-amino]carbonyl]-11H-dibenzo-[b,e][1,4]diazepin-11-on

5,10-Dihydro-5-[[N-methyl-(4-methyl-1-piperazinyl)amino]carbonyl]-11H-dibenzo[b,e][1,4]diazepin-11-on

Ein weiterer Gegenstand der Erfindung sind :

b) Arzneimittel, die ein oder mehrere Dibenzodiazepinone der allgemeinen Formel I bzw. deren physiologisch verträgliche Salze enthalten.

Die Verbindungen der allgemeinen Formel I lassen sich hierzu in an sich bekannter Weise in die üblichen pharmazeutischen Zubereitungsformen, z. B. in Lösungen, Suppositorien, Tabletten, Dragees, Kapseln oder Teezubereitungen einarbeiten. Die Tagesdosis liegt im allgemeinen zwischen 0,01 und 5, vorzugsweise 0,05 und 2,5, insbesondere 0,1 und 1,5 mg/kg Körpergewicht, die gegebenenfalls in Form mehrerer, vorzugsweise 1 bis 3 Einzelgaben zur Erzielung der gewünschten Ergebnisse verabreicht wird.

Die substituierten Dibenzodiazepinone der allgemeinen Formel I und ihre Säureadditionssalze besitzen wertvolle Eigenschaften, die sie gewerblich verwertbar machen, insbesondere sind sie durch eine ausgezeichnete Magen- und Darmschutzwirkung bei Warmblütern gekennzeichnet ; sie hemmen z. B. die Bildung von Magengeschwüren. Ferner weisen sie, bedingt durch eine geringe Toxizität und das Fehlen wesentlicher Nebenwirkungen eine günstige therapeutische Breite auf.

Die ausgezeichnete Wirksamkeit der substituierten Dibenzodiazepinone der allgemeinen Formel I und ihrer pharmakologisch, d. h. biologisch verträglichen Säureadditionssalze ermöglicht ihren Einsatz in der Human- und auch in der Veterinärmedizin, wobei sie zur Behandlung und Prophylaxe von Krankheiten, die auf Erkrankungen des Magens oder Darms beruhen, verwendet werden. Beispielsweise können mit ihnen akute und chronische Ulcera ventriculi und Ulcera duodeni, Gastritis und hyperacider Reizmagen bei Mensch und Tier behandelt werden.

Sollen die erfindungsgemäßen substituierten Dibenzodiazepinone der allgemeinen Formel I und/oder ihre pharmakologisch verträglichen Säureadditionssalze zur Behandlung der angegebenen Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxid, Magnesiumaluminat ; Sekretionshemmer, wie $H_2$-Blocker, z. B. Cimetidin, Ranitidin ; Magen- und Darmtherapeutika, z. B. Metoclopramid, Bromoprid, Tiaprid ; Tranquilizer, wie Benzodiazepine, beispielsweise Diazepam, Oxazepam ; Spasmolytika, z. B. Bietamiverin, Camylofin ; Anticholinergica, z. B. Oxyphencyclimin, Phencarbamid ; Glucocorticoide, wie Prednisolon, Fluocortolon, Betamethason ; nichtsteroidale Antiphlogistika, wie Arylessigsäuren und -propionsäuren, Heteroarylessigsäuren und -propionsäuren, Benzothiazincarboxamiddioxide, Pyrazolidindione, Chinazolinone, z. B. Ibuprofen, Naproxen, Diclofenac, Fenbufen, Flurbiprofen, Indometacin, Lonazolac, Sudoxicam, Piroxicam, Phenylbutazon, Bumadizon-Calcium, Proquazon ; Lokalanaesthetika, beispielsweise Tetracain, Procain ; gegebenenfalls auch Fermente, Vitamine, Aminosäuren etc. enthalten.

Ein weiterer Gegenstand der Erfindung sind

c) Verfahren zur Herstellung der substituierten Dibenzodiazepinone der allgemeinen Formel I

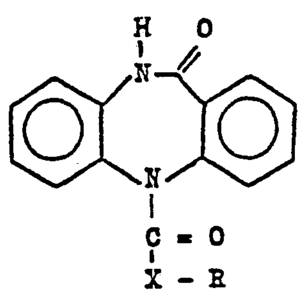

(I)

mit den eingangs erwähnten Bedeutungen für X und R, sowie ihrer Säureadditionssalze.

Die Verbindungen der allgemeinen Formel I lassen sich wie folgt herstellen :

a) Dibenzodiazepinone der allgemeinen Formel Ia

(Siehe Schema Seite 4 f.)

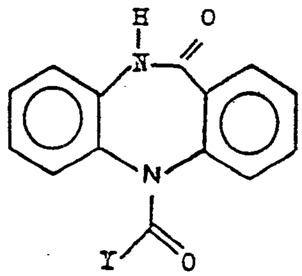

(Ia)

worin Y ein Halogenatom, bevorzugt ein Brom- oder Chloratom, oder den Rest $OR_1$ bedeutet, wobei $R_1$ einen gegebenenfalls halogensubstituierten Alkylrest mit 1 bis 5 Kohlenstoffatomen, einen gegebenenfalls durch Halogen oder Nitro substituierten Phenylrest oder einen Aralkylrest mit 7 bis 15 Kohlenstoffatomen darstellt ($R_1$ kann beispielsweise die Bedeutung eines Methyl-, Ethyl-, n-Butyl-, Isobutyl-, Benzyl-, 9-Fluorenylmethyl-, Phenyl-, 4-Nitrophenyl-, 2,2,2-Trichlorethyl-, 2,4,5-Trichlorphenyl- oder 2,2,2-Trichlor-tert.butyl-Restes annehmen), werden mit Verbindungen der allgemeinen Formel II

$$H—X—R \qquad (II)$$

in der X und R die eingangs angegebenen Bedeutungen haben, umgesetzt. Die Umsetzung wird ohne oder bevorzugt in Gegenwart inerter Lösungsmittel, z. B. von Wasser, Toluol oder von Alkoholen, wie z. B. Methanol, Ethanol oder Isopropanol, ganz bevorzugt jedoch in Gegenwart aprotischer polarer Lösungsmittel, z. B. von Tetrahydrofuran, 1,4-Dioxan, Acetonitril, N,N-Dimethylformamid, Dimethylsulfoxid, Hexamethylphosphorsäuretriamid, oder von Gemischen davon und bei Temperaturen zwischen 0 °C und dem Siedepunkt des Reaktionsgemisches, bevorzugt zwischen 40 und 100 °C durchgeführt. Bewährt hat sich die Verwendung zusätzlicher anorganischer oder organischer Basen, z. B. von Alkali- oder Erdalkalihydroxiden, -alkoholaten oder -carbonaten, etwa von Natriumhydroxid, Natriummethanolat, Kalium-tert.butanolat, Natriumcarbonat, Kaliumcarbonat ; von tertiären Aminen, z. B. Triethylamin, Ethyldiisopropylamin, N,N-Dimethylanilin oder von Pyridin ; sowie die Umsetzung in Gegenwart eines Überschusses einer Verbindung der allgemeinen Formel II.

Gute Ergebnisse erzielt man auch durch Umsetzung eines Dibenzodiazepinons der allgemeinen Formel Ia mit einer Metallverbindung der allgemeinen Formel IIa

$$M—X—R \qquad (IIa)$$

in der M ein Alkalimetallatom oder 1 Äquivalent eines Erdalkalimetallatoms bedeutet. Dabei lassen sich Metallverbindungen der allgemeinen Formel IIa aus II durch Umsetzung mit Alkali- oder Erdalkalimetallen, etwa mit Natrium, Kalium oder Barium, oder mit Alkali- oder Erdalkalihydriden, etwa mit Natrium-, Kalium- oder Calciumhydrid, oder durch Reaktion mit Alkali- oder Erdalkaliorganylen, z. B. mit n-Butyllithium oder Phenyllithium, in situ leicht herstellen.

b) Durch Umsetzung von 5,10-Dihydro-11H-dibenzo[b,e][1,4]-diazepin-11-on mit Chlorkohlensäurederivaten der allgemeinen Formel IV

$$Cl - \underset{\underset{\displaystyle O}{\|}}{C} - X - R \qquad (IV)$$

oder mit Isocyanaten der allgemeinen Formel IVa

$$O = C = N—R \qquad (IVa)$$

worin R und X wie oben definiert sind, erhält man ebenfalls die erfindungsgemäßenen Verbindungen der allgemeinen Formel I. Diese Umsetzung wird in inerten organischen Lösungsmitteln, beispielsweise in aromatischen Kohlenwasserstoffen wie Toluol, Xylol, in Äthern wie Diisopropyläther, Tetrahydrofuran oder Dioxan, in Ketonen wie 3-Pentanon, in chlorierten aliphatischen Kohlenwasserstoffen, wie 1,2-Dichlorethan oder in anderen Lösungsmitteln, wie Acetonitril oder Dimethylformamid oder in Gemischen davon, gegebenenfalls in Gegenwart tertiärer organischer Basen, wie Pyridin, und bei Temperaturen bis zum Siedepunkt des Reaktionsgemisches, bevorzugt bei Temperaturen zwischen + 30 und + 100 °C durchgeführt.

c) Durch Umsetzung von 5,10-Dihydro-11H-dibenzo[b-e][1,4]-diazepin-11-on mit einem Lithiumalkyl oder -aryl zu dessen Di-Lithiumsalz und anschließende Umsetzung des Di-Lithiumsalzes mit einer Verbindung der allgemeinen Formel V

$$R_2O - \overset{\overset{\displaystyle O}{\|}}{C} - X - R \qquad (V)$$

in der X und R wie oben definiert sind und $R_2$ einen Alkylrest mit 1 bis 10 Kohlenstoffatomen, vorzugsweise 1 bis 6 Kohlenstoffatomen oder einen Aralkylrest mit 7 bis 13 Kohlenstoffatomen, vorzugsweise den Phenylmethyl-, Phenylethyl- oder Phenylpropylrest bedeutet. X stellt vorzugsweise ein Sauerstoffatom dar.

Die Überführung des 5,10-Dihydro-11H-dibenzo[b,e][1,4]-diazepin-11-ons in sein Di-Lithiumsalz gelingt mit Lithiumalkylen, insbesondere aber mit n-Butyllithium, n-Butyllithium in Gegenwart von Tetramethylethylendiamin, tertiär-Butyllithium, Lithiumdiisopropylamid, Lithiumdicyclohexylamid oder mit Lithiumarylen, z. B. mit Lithiumphenyl. Die Überführung in das Lithiumsalz und die weitere Umsetzung mit einer Verbindung der allgemeinen Formel V erfolgt in einem organischen Lösungsmittel bei Temperaturen zwischen − 60 und 0 °C, vorzugsweise aber bei − 10 °C. Als organische Lösungsmittel dienen solche, die für Umsetzungen mit Lithiumalkylen oder Lithiumarylen gebräuchlich sind, beispielsweise Tetrahydrofuran oder Ether, wie Diethylether, aliphatische Kohlenwasserstoffe, wie Hexan oder Gemische dieser Lösungsmittel ; gegebenenfalls kann auch ein Colsovens, z. B. Hexamethylphosphoramid, zugegen sein. Kurze Zeit nach der Beendigung der Zugabe des Lithiumalkyls oder -aryls gibt man die stöchiometrische Menge oder einen leichten Überschuß des Esters der allgemeinen Formel V zu und läßt das Reaktionsgemisch zur Vervollständigung der Reaktion langsam, z. B. innerhalb von 2 Stunden, auf Raumtemperatur kommen. Die nach üblichen Methoden isolierte Verbindung der allgemeinen Formel I kann anschließend in ihre Salze überführt werden.

So erhaltene Basen der allgemeinen Formel I können anschließend in ihre Säureadditionssalze oder erhaltene Säureadditionssalze in die freien Basen oder andere pharmakologisch verträgliche Säureadditionssalze übergeführt werden.

Ein Teil der erfindungsgemäßen Dibenzodiazepinone der allgemeinen Formel I enthält ein oder zwei asymmetrische Kohlenstoffatome in der Seitenkette —CO—X—R. Diese Verbindungen können deshalb in zwei diastereomeren cis- und trans-Formen oder jeweils als enantiomere (+)- und (−)-Formen auftreten. Die Erfindung umfaßt die einzelnen Isomeren ebenso wie ihre Gemische.

Die Trennung der jeweiligen Diastereomeren gelingt auf Grund der unterschiedlichen physikochemischen Eigenschaften, z. B. durch fraktioniertes Umkristallisieren aus geeigneten Lösungsmitteln, durch Hochdruckflüssigkeitschromatographie oder gaschromatographische Verfahren. Jeweils nur ein Diastereomeres wird erhalten, wenn man die oben beschriebenen Synthesen mit nur einem Diastereomeren der allgemeinen Formel II bzw. IIa durchführt.

Die Spaltung evtl. Razemate der Verbindungen der allgemeinen Formel I kann nach bekannten Verfahren durchgeführt werden, beispielsweise unter Verwendung einer optisch aktiven Säure, wie (+)- oder (−)-Weinsäure, oder eines Derivats davon, wie (+)- oder (−)-Diacetylweinsäure, (+)- oder (−)-Monomethyltartrat oder (+)-Camphersulfonsäure.

Nach einem üblichen Verfahren zur Isomerentrennung wird das Razemat einer Verbindung der allgemeinen Formel I mit einer der vorstehend beschriebenen optisch aktiven Säuren in äquimolarer Menge in einem Lösungsmittel umgesetzt und die erhaltenen kristallinen optisch aktiven Salze werden unter Ausnutzung ihrer verschiedenen Löslichkeit getrennt. Diese Umsetzung kann in jeder Art von Lösungsmittel durchgeführt werden, solange dieses einen ausreichenden Unterschied in der Löslichkeit der Salze aufweist. Vorzugsweise werden Methanol, Ethanol oder deren Gemische, beispielsweise im Volumenverhältnis 50 : 50, verwendet. Sodann wird jedes der optisch aktiven Salze in Wasser gelöst, mit einer Base, wie Natriumcarbonat oder Kaliumcarbonat, neutralisiert und dadurch die entsprechende freie Verbindung in der (+)- oder (−)-Form erhalten.

Jeweils nur ein Enantiomeres wird auch dadurch erhalten, daß man die oben beschriebenen Synthesen mit nur einem Enantiomeren der allgemeinen Formel II bzw. IIa durchführt.

Die als Ausgangsmaterialien erforderlichen Verbindungen der allgemeinen Formel Ia erhält man durch Umsetzung des literaturbekannten 5,10-Dihydro-11H-dibenzo[b,e][1,4]diazepin-11-on mit Halogenkohlensäurederivaten der allgemeinen Formel III

$$\mathrm{Hal} - \underset{\underset{\displaystyle O}{\|}}{C} - Y \qquad \text{(III)}$$

in der Hal ein Brom-, bevorzugt ein Chloratom bedeutet und Y die oben angegebenen Bedeutungen hat. Die Reaktion wird in inerten organischen Lösungsmitteln, beispielsweise aromatischen Kohlenwasserstoffen, wie Toluol, Chlorbenzol oder Xylol ; offenkettigen oder cyclischen Ethern, wie Diisopropylether, Tetrahydrofuran oder Dioxan ; offenkettigen oder cyclischen aliphatischen Ketonen, beispielsweise 3-Pentanon ; chlorierten aliphatischen Kohlenwasserstoffen, wie 1,2-Dichlorethan oder anderen Lösungsmitteln, wie Acetonitril oder Dimethylformamid oder in Gemischen davon und bevorzugt in Gegenwart tertiärer organischer Basen, bevorzugt von Pyridin, und bei Temperaturen bis höchstens zum Siedepunkt des verwendeten Lösungsmittels oder Lösungsmittelgemisches, bevorzugt zwischen + 30 und + 80 °C, durchgeführt.

So wurde beispielsweise erhalten :

5-Chlorcarbonyl-5,10-dihydro-11H-dibenzo[b,e][1,4]diazepin-11-on, F. 267-268 °C (Z.).

Die Ausgangsverbindungen der allgemeinen Formel II sind bekannt oder können in Analogie zu literaturbekannten Verfahren bereitet werden. Beispielsweise erhält man 1-Hydroxy-4-methyl-piperazin nach S. M. Riba, A. S. Issa und Y. A. Beltagy, Pharmazie *33,* 711 (1978) durch Umsetzung von Bis [N-(2-Chlorethyl)] methylamin mit Hydroxylaminhydrochlorid in wäßrigethanolischer Lösung und in Gegenwart von Kaliumcarbonat, 3 α-Aminotropan und 3 α-Methylaminotropan nach S. Archer u. a., J. Amer. Chem. Soc. *79,* 4194-4198 (1957) ; 3ß-Aminotropan nach R. Willstätter u. a., Ber. dtsch. chem. Ges. *31,* 1202 (1898) ; Pseudotropin nach J. J. Tufariello u. a., J. Amer. Chem. Soc. *101,* 2435-2442 (1979).

Halogenkohlensäurederivate der allgemeinen Formel III sind bekannt.

Chlorkohlensäurederivate der allgemeinen Formel IV und Isocyanate der allgemeinen Formel IVa sind bekannt oder können in Analogie zu literaturbekannten Verfahren erhalten werden (S. z. B. I. W. Mathison u. a., J. Pharm. Sci. *62,* 158 [1963] ; H. Hopff und H. Ohlinger, Angew. Chem. *61,* 183 [1949] ; W. Siefken, Liebigs Ann. Chem. *562,* 75 [1949] ; Houben-Weyl VIII, 117 ; Ullmann V, 72 ; L. C. Raiford und K. Alexander, J. Org. Chem. *5,* 306 [1940] ; H. H. Saunders und R. J. Slocombe, Chem. Rev. *43,* 203 [1948] ; R. J. Slocombe, E. E. Hardy, J. H. Saunders und R. L. Jenkins, J. Amer. chem. Soc. *72,* 1888 [1950] ; H. Habad und A. G. Zeiler, Chem. Rev. *73,* 75 [1973]).

Die Ester der allgemeinen Formel V sind literaturbekannt oder lassen sich in Anlehnung an literaturbekannte Methoden herstellen.

Wie bereits oben erwähnt, weisen die neuen Verbindungen der allgemeinen Formel I wertvolle pharmakologische Eigenschaften auf ; insbesondere besitzen sie antiulcerogene und magensäuresekretionshemmende Wirkungen, sowie günstige Effekte auf verschiedene andere Erkrankungen des Magen-Darm-Traktes, unter denen das Colon-irritabile besonders hervorgehoben sei.

Eine günstige Relation zwischen antiulcerogenen und antisekretorischen Wirkungen einerseits und den vor allem bei Therapeutika mit anticholinerger Wirkkomponente auftretenden unerwünschten Wirkungen auf die Pupillenweite und Tränen- und Speichelsekretion andererseits ist für die therapeutische Verwendung der Substanzen von besonderer Wichtigkeit. Die folgenden Versuche zeigen, daß die erfindungsgemäßen Verbindungen diesbezüglich überraschend günstige Relationen aufweisen.

Untersuchung auf Selektivität der antimuscarinischen Wirkung

Ziel :

Oxotremorin, ein spezifischer Agonist an muscarinischen Rezeptoren, erzeugt bei Ratten Läsionen der Magenschleimhaut und steigert die Speichelsekretion. Dieses Versuchsmodell wurde gewählt, um eine selektive Wirkung einer antimuscarinischen Substanz auf den Magen erkennen zu können.

Methodik :

Verwendet wurden 10 weibliche Albino-Ratten (Stamm Crl : COBS-CD (SD) BR) pro Behandlungsgruppe mit einem Körpergewicht von 120 bis 150 g, die bei freiem Zugang zu Trinkwasser 24 Stunden vor Versuchsbeginn ohne Nahrung waren.

Um in Vorversuchen die muscarinische Wirkung von Oxotremorin auf jedes der untersuchten Symptome zu ermitteln, wurde mit jeweils mindestens drei Dosen für jedes Symptom eine Dosis-Wirkungskurve erstellt.

Bei der Prüfung antimuscarinischer Substanzen wurde die Oxotremorin-Dosis gewählt, die in den Vorversuchen das zu beeinflussende Symptom bei 90 bis 100 % der Tiere ausgelöst hatte.

| | |
|---|---|
| Magenschleimhaut-Läsionen : | 0,62 mg/kg i. v. |
| Speichelsekretion : | 0,083 mg/kg i. v. |

Jede antimuscarinische Substanz wurde in gleichmäßig abgestuften Dosierungen 15 Minuten vor der Oxotremorin-Gabe intravenös verabreicht. Kontrollgruppen erhielten an Stelle der Prüfsubstanz das Lösungs- und Suspensionsmittel in entsprechender Menge.

Unmittelbar nach Oxotremorin-Gabe wurden die Tiere in einem Glaskäfig für 15 Minuten beobachtet.

Die Prüfung auf Beeinflussung der Oxotremorin-induzierten Speichelsekretion wurde blind durchgeführt, d. h. der Untersucher wußte nicht, wie die Tiere vorbehandelt waren.

Die Ergebnisse wurden als prozentuale Hemmung des Oxotremorin-Effektes (Prozentsatz der Tiere ohne das entsprechende Symptom) ausgedrückt. $ED_{50}$-Werte wurden nach der Methode von LITCHFIELD und WILCOXON (J. Pharmacol. Exp. Ther. *96,* 99, 1949) ermittelt.

Die Effekte auf Schleimhaut-Läsionen des Magens wurden folgendermaßen bewertet :

Die Magenschleimhaut-Läsionen wurden erzeugt durch intravenöse Injektion von 0,62 mg/kg Oxotremorin 30 Minuten nach oraler Gabe von 1 mg/kg Neostigmin (Cholinesterase-Hemmer). 60 Minuten nach Neostigmin-Gabe wurden die Tiere getötet, die Mägen entnommen, geöffnet und auf Vorhandensein von Schleimhaut-Läsionen geprüft. Die schützende Wirkung der Prüfsubstanzen wurde als prozentuale Hemmung (Prozentsatz der Tiere ohne Läsionen) ausgedrückt. $ED_{50}$- bzw. $ED_{70}$-Werte wurden nach der Methode von LITCHFIELD und WILCOXON (s. o.) ermittelt.

Mydriasis

Der Effekt von Testsubstanzen auf die Pupillenweite von Ratten wurde folgendermaßen untersucht :

Die Substanzen wurden an Gruppen von jeweils 10 Tieren in wenigstens 3 gleichmäßig abgestuften Dosierungen intravenös appliziert. Die Pupillenweite wurde anschließend 10 Minuten lang auf evtl. Veränderungen (Auftreten von Mydriasis oder Miosis) beobachtet, und zwar wiederum blind, d. h. dem jeweiligen Untersucher war die Vorbehandlung der Tiere unbekannt. Es wurde der Prozentsatz der Versuchstiere ermittelt, bei denen eine Mydriasis auftrat. $ED_{50}$-Werte wurden wiederum nach LITCHFIELD und WILCOXON (s. o.) bestimmt.

2. Bindungsstudien an muscarinischen Rezeptoren : Bestimmung des $IC_{50}$-Wertes

Als Organspender dienten männliche Sprague-Dawley-Ratten mit 180-220 g Körpergewicht. Nach Entnahme von Herz, Magen und Großhirnrinde wurden alle weiteren Schritte im eiskalten Hepes-HCl-Puffer (pH 7,4 ; 100 m molar NaCl, 10 m molar $MgCl_2$) vorgenommen. Die glatte Muskulatur des Magenfundus wurde von der Magenschleimhaut freipräpariert und vorhomogenisiert. Das Gesamtherz wurde mit einer Schere zerkleinert. Alle Organe wurden abschließend in einem Potter homogenisiert.

Für den Bindungstest wurden die Organhomogenate in folgender Weise verdünnt :

| | |
|---|---|
| Glatter Muskel Magenfundus | 1 : 100 |
| Gesamtherz | 1 : 250 |
| Großhirnrinde | 1 : 3 000 |

Die Inkubation der Organhomogenate erfolgte bei einer bestimmten Konzentration des Radioliganden und einer Konzentrationsreihe der nichtradioaktiven Testsubstanzen im Eppendorf-Zentrifugenröhrchen bei 30 °C. Die Inkubationsdauer betrug 45 Minuten. Als Radioligand wurde 0,3 n molar $^3H$-N-Methylscopolamin ($^3H$-NMS) verwendet. Nach Beendigung der Inkubation durch Zentrifugation bei 14 000 g wurde die Radioaktivität im Pellet bestimmt. Sie repräsentiert die Summe von spezifischer und unspezifischer Bindung von $^3H$-NMS. Der Anteil der unspezifischen Bindung wurde definiert als jene Radioaktivität, die in Anwesenheit von 1 $\mu$ molar Chinuclidinylbenzilat gebunden wurde. Es wurden immer Vierfachbestimmungen vorgenommen. Die $IC_{50}$-Werte der nichtmarkierten Testsubstanzen wurden graphisch bestimmt. Sie repräsentieren jene Konzentration der Testsubstanz, bei welcher die spezifische Bindung von $^3H$-NMS an die muscarinischen Rezeptoren in den verschiedenen Organen um 50 % gehemmt wurde.

Nach den vorstehenden Angaben wurden beispielsweise die folgenden Verbindungen untersucht :

A = 5,10-Dihydro-5-[[(1-methyl-4-piperidinyl)oxy]carbonyl]-11H-dibenzo[b,e][1,4]diazepin-11-on
B = 5,10-Dihydro-5-[[(1-methyl-4-piperidinyl)amino]carbonyl]-11H-dibenzo[b,e][1,4]diazepin-11-on
C = 5,10-Dihydro-5-[[(4-methyl-1-piperazinyl)amino]carbonyl]-11H-dibenzo[b,e][1,4]diazepin-11-on

(Siehe Tabelle Seite 8 f.)

Ergebnisse :

| Substanz | Rezeptor-Bindungs-Tests $IC_{50}$ [n Mol l $^{-1}$] | | | Oxotremorin-Tests [µg/kg] i.v. | | | Mydriasis Pupillenweite (Ratte) |
|---|---|---|---|---|---|---|---|
| | Cortex | Glatter Muskel Magenfundus | Herz | Antiulcerogene Wirkung $ED_{50}$ | Antiulcerogene Wirkung $ED_{70}$ | Salivationshemmung $ED_{50}$ | $ED_{50}$ i.v. [µg/kg] |
| A | 5,5 | 60 | 42 | 15,5 | 36 | 64 | 71 |
| B | 13 | 100 | 75 | 2,5 | 4,8 | 22 | 23 |
| C | 18 | 90 | | 3,6 | 7 | 32 | 82,5 |

Die Angaben in der vorstehenden Tabelle beweisen, daß die genannten Verbindungen generell eine hohe Affinität zu muscarinischen Rezeptoren besitzen. Darüber hinaus kann man den Daten entnehmen, daß die neuen Verbindungen der allgemeinen Formel I zwischen muscarinischen Rezeptoren verschiedener Gewebe unterscheiden. Dies folgt aus den beträchtlich niedrigeren $IC_{50}$-Werten bei Untersuchung an Präparaten aus der Großhirnrinde gegenüber solchen aus der glatten Muskulatur von Magen und Herz.

Aus den pharmakologischen Daten der vorstehenden Tabelle ergibt sich — in völliger Übereinstimmung mit den Rezeptor-Bindungs-Studien —, daß die Entstehung Oxotremorin-induzierter Magenschleimhaut-Läsionen durch die genannten Verbindungen bereits bei Dosierungen gehemmt wird, bei denen noch keine Einschränkung der Speichelsekretion und keine Mydriasis beobachtet wird.

Die folgenden Beispiele dienen zur näheren Erläuterung der Erfindung. « F. » bedeutet « Schmelzpunkt », « Z. » bedeutet « Zersetzung ».

## Beispiel 1

5,10-Dihydro-5-[[(1-methyl-4-piperidinyl)amino]carbonyl]-11H-dibenzo[b,e][1,4]diazepin-11-on

3,2 g (0,012 Mol) 5-Chlorcarbonyl-5,10-dihydro-11H-dibenzo-[b,e][1,4]diazepin-11-on und 2,1 g (0,02 Mol) Natriumcarbonat wurden in 100 ml Acetonitril zusammen mit 2,3 ml (0,02 Mol) 4-Amino-1-methylpiperidin × 15 Minuten unter Rückfluß gerührt. Es wurde heiß filtriert, das Filtrat wurde über eine Kieselgelsäule gereinigt (Fließmittel : Acetonitril ; anschließend Methylenchlorid + Essigsäureethylester + Cyclohexan + Methanol + Ammoniak = 175 +75 +23 + 23 + 3). Die gewünschte Fraktion wurde im Vakuum eingedampft, der Rückstand wurde aus 50-prozentigem wässrigem Acetonitril umkristallisiert.

F. = 128-130 °C. Ausbeute : 56 % der Theorie.

Entsprechend erhielt man :

cis-5,10-Dihydro-5-[[1,2-dimethyl-4-piperidinyl)amino]carbonyl]-11H-dibenzo[b,e][1,4]diazepin-11-on

trans-5,10-Dihydro-5-[[1,2-dimethyl-4-piperidinyl)amino]-carbonyl]-11H-dibenzo[b,e][1,4] diazepin-11-on

cis-5,10-Dihydro-5-[[1,3-dimethyl-4-piperidinyl)amino]-carbonyl]-11H-dibenzo[b,e][1,4]diazepin-11-on

trans-5,10-Dihydro-5-[[1,3-dimethyl-4-piperidinyl)amino]-carbonyl]-11H-dibenzo[b,e][1,4]diazepin-11-on

exo-5,10-Dihydro-5-[[(8-methyl-8-azabicyclo[3,2,1]oct-3-yl)-amino]carbonyl]-11H-dibenzo[b,e][1,4]diazepin-11-on, F. 209-211 °C (3-Pentanon).

## Beispiel 2

5,10-Dihydro-5-[[N-methyl-N-(1-methyl-4-piperidinyl)amino]-carbonyl]-11H-dibenzo[b,e][1,4]diazepin-11-on

9,0 g (0,033 Mol) 5-Chlorcaarbonyl-5,10-dihydro-11H-dibenzo-[b,e][1,4]diazepin-11-on, 5,3 g (0,05 Mol) Natriumcarbonat und 5,2 g (0,036 Mol) 1-Methyl-4-methylamino-piperidin wurden in 150 ml Acetonitril 15 Minuten unter Rückfluß gerührt. Es wurde heiß filtriert, das Filtrat wurde auf eine Kieselgelsäule gegeben und mit Acetonitril und anschließend mit einem Gemisch von Methylenchlorid + Essigsäureethylester + Cyclohexan + Methanol + Ammoniak = 175 + 75 + 23 + 23 + 3 eluiert. Die gewünschte Fraktion wurde im Vakuum eingedampft, der Rückstand wurde aus Acetonitril umkristallisiert.

F. = 232-234 °C. Ausbeute : 62 % der Theorie.

cis-5,10-Dihydro-5-[[N-methyl-N-(1,2-dimethyl-4-piperidinyl)-amino]carbonyl]-11H-dibenzo[b,e][1,4]diazepin-11-on

trans-5,10-Dihydro-5-[[N-methyl-N-(1,2-dimethyl-4-piperidinyl)-amino]carbonyl]-11H-dibenzo[b,e][1,4] diazepin-11-on

cis-5,10-Dihydro-5-[[N-methyl-N-(1,3-dimethyl-4-piperidinyl)-amino]carbonyl]-11H-dibenzo[b,e][1,4]diazepin-11-on

trans-5,10-Dihydro-5-[[N-methyl-N-(1,3-dimethyl-4-piperidinyl)-amino]carbonyl]-11H-dibenzo[b,e][1,4] diazepin-11-on

endo-5,10-Dihydro-5-[[N-methyl-N-(8-methyl-8-azabicyclo[3,2,1]-oct-3-yl)amino]carbonyl]-11H-dibenzo [b,e][1,4]diazepin-11-on, F. 242-245 °C (Acetonitril),

exo-5,10-Dihydro-5-[[N-methyl-N-(8-methyl-8-azabicyclo[3,2,1]-oct-3-yl)amino]carbonyl]-11H-dibenzo [b,e][1,4]diazepin-11-on

5,10-Dihydro-5-[[N-methyl-(4-methyl-1-piperazinyl)amino]-carbonyl]-11H-dibenzo[b,e][1,4]diazepin-11-on, F. 297 °C (Methanol/Essigsäureethylester 1 : 2).

## Beispiel 3

5,10-Dihydro-5-[[(4-methyl-1-piperazinyl)amino]carbonyl]-11H-dibenzo[b,e][1,4]diazepin-11-on

Die Mischung aus 5,5 g (0,0202 Mol) 5-Chlorcarbonyl-5,10-dihydro-11H-dibenzo[b,e][1,4]diazepin-11-

on, 8,6 g (0,075 Mol) 1-Amino-4-methylpiperazin und 200 ml wasserfreiem Dioxan wurde 30 Minuten auf dem Dampfbad erwärmt, die erhaltene trübe Reaktionsmischung noch heiß mit 2 g Aktivkohle versetzt, filtriert und das erhaltene Filtrat im Vakuum eingeengt. Der Rückstand wurde an 300 g Kieselgel unter Verwendung einer Mischung aus Dichlormethan, Methanol und konz. wäßrigem Ammoniak (Volumenverhältnis 800 : 200 : 5) säulenchromatographisch gereinigt. Der nach dem Eindampfen der Eluate verbleibende Rückstand wurde in 500 ml Essigsäureethylester aufgenommen, nach Zusatz von 1 g Aktivkohle aufgekocht, filtriert und eingeengt, nach Zugabe von 300 ml Methanol abermals heiß filtriert und zur Trockne gedampft. Nach dem Umkristallisieren aus Ethanol erhielt man 1,8 g (25 % der Theorie) an farblosen Kristallen vom F. 213-214 °C (Z.).

Entsprechend erhielt man :

5,10-Dihydro-5-[[(3,4-dimethyl-1-piperazinyl)amino]carbonyl]-11H-dibenzo[b,e][1,4]diazepin-11-on

5,10-Dihydro-5-[[(2,4-dimethyl-1-piperazinyl)amino]carbonyl]-11H-dibenzo[b,e][1,4]diazepin-11-on

endo-5,10-Dihydro-5-[[N-methyl-N-(8-methyl-8-azabicyclo[3,2,1]-oct-3-yl)amino]carbonyl]-11H-dibenzo [b,e][1,4]diazepin-11-on, F. 242-245 °C (Acetonitril),

5,10-Dihydro-5-[[N-methyl-N-(1-methyl-4-piperidinyl)amino]-carbonyl]-11H-dibenzo[b,e][1,4]diazepin-11-on, F. 232-233 °C (Acetonitril).

## Beispiel 4

5,10-Dihydro-5-[[(1-methyl-4-piperidinyl)oxy]carbonyl]-11H-dibenzo[b,e][1,4]diazepin-11-on

4,1 g (0,015 Mol) 5-Chlorcarbonyl-5,10-dihydro-11H-dibenzo-[b,e][1,4]diazepin-11-on wurden mit 3,45 g (0,03 Mol) 1-Methyl-4-piperidinol in 50 ml Chlorbenzol zwei Stunden unter Rückfluß erhitzt. Nach dem Abkühlen wurde die Reaktionslösung durch Zugabe von 200 ml Essigsäureethylester verdünnt und anschließend mit 15 prozentiger Salzsäure erschöpfend extrahiert. Man neutralisierte die vereinigten Extrakte mit Kaliumcarbonat, extrahierte mehrmals mit Chloroform und engte die vereinigten Chloroformauszüge im Vakuum zur Trockne ein. Durch Umkristallisation aus Essigsäureethylester erhielt man farblose Kristalle vom F. 203-204 °C. Ausbeute : 1,2 g (23 % der Theorie).

Entsprechend erhielt man :

cis-5,10-Dihydro-5-[[(1,2-dimethyl-4-piperidinyl)oxy]carbonyl]-11H-dibenzo[b,e][1,4]diazepin-11-on

trans-5,10-Dihydro-5-[[(1,2-dimethyl-4-piperidinyl)oxy]carbonyl]-11H-dibenzo[b,e][1,4]diazepin-11-on

cis-5,10-Dihydro-5-[[(1,3-dimethyl-4-piperidinyl)oxy]carbonyl]-11H-dibenzo[b,e][1,4]diazepin-11-on

trans-5,10-Dihydro-5-[[(1,3-dimethyl-4-piperidinyl)oxy]carbonyl]-11H-dibenzo[b,e][1,4]diazepin-11-on

5,10-Dihydro-5-[[(4-methyl-1-piperazinyl)oxy]carbonyl]-11H-dibenzo[b,e][1,4]diazepin-11-on

5,10-Dihydro-5-[[3,4-dimethyl-1-piperazinyl)oxy]carbonyl]-11H-dibenzo[b,e][1,4]diazepin-11-on

5,10-Dihydro-5-[[(2,4-dimethyl-1-piperazinyl)oxy]carbonyl]-11H-dibenzo[b,e][1,4]diazepin-11-on

endo-5,10-Dihydro-5-[[(8-methyl-8-azabicyclo[3,2,1]oct-3-yl)-oxy]carbonyl]-11H-dibenzo[b,e][1,4]diazepin-11-on, $R_F$-Wert 0,55 (Fließmittel Methylenchlorid/Cyclohexan/Methanol/Ammoniak 68 : 15 : 15 : 2, Kieselgelfertigplatte 60 $F_{254}$)

exo-5,10-Dihydro-5-[[(8-methyl-8-azabicyclo[3,2,1]oct-3-yl)-oxy]carbonyl]-11H-dibenzo[b,e][1,4] diazepin-11-on

5,10-Dihydro-5-[[(4-methyl-4-piperidinyl)oxy]carbonyl]-11H-dibenzo[b,e][1,4]diazepin-11-on, F.178-179 °C (Diethylether).

## Beispiel 5

5,10-Dihydro-5-[[(1-methyl-4-piperidinyl)oxy]carbonyl]-11H-dibenzo[b,e][1,4]diazepin-11-on

In eine Mischung, bestehend aus 22,5 ml einer 20 prozentigen Lösung von Phosgen in Toluol, 100 ml Dioxan und 4,75 g (0,045 Mol) wasserfreiem Natriumcarbonat wurden unter äußerer Kühlung mit Eis 4,9 g (0,0425) Mol) 1-Methyl-4-piperidinol zugetropft. Man rührte noch 60 Minuten bei Zimmertemperatur, trug dann 9,0 g (0,0428 Mol) 5,10-Dihydro-11H-dibenzo[b,e][1,4]diazepin-11-on in die Reaktionsmischung ein und kochte anschließend 4 Stunden unter Rückfluß. Man filtrierte, dampfte das Filtrat im Vakuum ein und reinigte das erhaltene Rohprodukt säulenchromatographisch an 500 g Kieselgel unter Verwendung von Essigsäureethylester/Methanol (Volumenverhältnis 10 : 1) zum Eluieren. Nach dem Umkristallisieren aus Essigsäureethylester schmolzen die farblosen Kristalle bei 203-204 °C und waren nach Mischschmelzpunkt, Dünnschichtchromatogramm und IR-Spektrum identisch mit einem nach Beispiel 4 hergestellten Präparat. Ausbeute : 7,6 g (51 % der Theorie).

Entsprechend erhielt man :

5,10-Dihydro-5-[[(4-methyl-1-piperazinyl)oxy]carbonyl]-11H-dibenzo[b,e][1,4]diazepin-11-on

endo-5,10-Dihydro-5-[[(8-methyl-8-azabicyclo[3,2,1]oct-3-yl)-oxy]carbonyl]-11H-dibenzo[b,e][1,4]diazepin-11-on, $R_F$-Wert : 0,55 (Kieselgelfertigplatte 60 $F_{254}$, Fließmittel Methylenchlorid/Cyclohexan/Methanol/Ammoniak 68 : 15 : 15 : 2).

Beispiel 6

endo-5,10-Dihydro-5-[[(8-methyl-8-azabicyclo[3,2,1]oct-3-yl)-amino]carbonyl]-11H-dibenzo[b,e][1,4]diazepin-11-on

Hergestellt analog Beispiel 1 aus 5-Chlorcarbonyl-5,10-dihydro-11H-dibenzo[b,e][1,4]diazepin-11-on und endo-3-Amino-8-methyl-8-azabicyclo[3,2,1]octan in einer Ausbeute von 58 % der Theorie. Farblose Kristalle vom F. 264-266 °C (Acetonitril).

$C_{22}H_{24}N_4O_2$ (376,46)
Ber. : C 70,19 H 6,43 N 14,88
Gef. : C 69,90 H 6,53 N 14,94
IR ($CH_2Cl_2$) : NH 3450/cm, 3370/cm ; CO 1660 und 1675/cm
UV (Ethanol) : Schulter bei λ 235 (E = 0,17) und λ 272 nm (E = 0,075)
(Ethanol/KOH) : Schulter bei λ 250 nm (E = 0,105) ; λ max 288 nm (E = 0,075)
(c = 50 mg/l ; Schichtdicke : 2 mm)
$^1$H-NMR ($CDCl_3$) :
δ = 9,35 (1H-s ; austauschbarer H) ; 7,96 (1H-dd ; J = 5 und 2 Hz ; ar. H) ; 7,0-7,7 (7H-m ; ar. H) ; 4,94 (1H-d ; J = 5 Hz ; austauschbarer H) ; 3,7-4,1 (1H-m) ; 2,8-3,15 (2H-m) ; 2,21 (3H-s ; N-$CH_3$) ; 1,0-2,2 ppm (8H-m).

Beispiel 7

5,10-Dihydro-5-[[(1-methyl-4-piperidinyl)oxy]carbonyl]-11H-dibenzo[b,e][1,4]diazepin-11-on

Zu einer Lösung von 5 g (0,024 Mol) 5,10-Dihydro-5H-dibenzo-[b,e][1,4]diazepin-11-on in 150 ml Tetrahydrofuran wurden bei + 10 °C, unter Rühren, 32 ml (0,05 Mol) N-Butyl-lithium (15 %ig in n-Hexan) eingetropft. Zusätzlich wurde noch 0,5 Stunden bei Raumtemperatur gerührt und dann eine Lösung von 5,6 g (0,03 Mol) 1-Methyl-piperidin-4-oxy-carbonsäure-ethylester in 25 ml trockenem Tetrahydrofuran — wieder bei 10 °C — zugetropft und dann 2 Stunden bei Raumtemperatur gerührt. Danach wurde das Reaktionsgemisch auf ca. 1/3 des Volumens im Vakuum eingedampft und mit Eis/Wasser zersetzt. Anschließend wurde dreimal mit Essigester ausgeschüttlet. Der wäßrige, saure Anteil wurde in der Kälte mit festem Kaliumkarbonat alkalisch gestellt und wieder dreimal mit Essigester extrahiert. Diese Extrakte wurden getrocknet und im Vakuum eingedampft. Der Rückstand wurde aus Ethanol umkristallisiert und ergab 3,4 g (40 % der Theorie) 5,10-Dihydro-5-[[(1-methyl-4-piperidinyl)oxy]carbonyl]-11H-dibenzo[b,e][1,4]diazepin-11-on, F. 203-204 °C, das in seinen physikalischen Eigenschaften mit dem nach Beispiel 4 erhaltenen Produkt identisch ist.

Im folgenden wird die Herstellung pharmazeutischer Anwendungsformen anhand einiger Beispiele beschrieben :

Beispiel I

Tabletten mit 5 mg 5,10-Dihydro-5-[[(1-methyl-4-piperidinyl)-amino]carbonyl]-11H-dibenzo[b,e][1,4]diazepin-11-on

Zusammensetzung :
1 Tablette enthält :

| | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren :

Aus Kartoffelstärke wird durch Erwärmen ein 10 %iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45 °C getrocknet, nochmals durch obiges Sieb gerieben, mit Magnesiumstearat vermischt und zu Tabletten verpreßt.

Tablettengewicht : 220 mg
Stempel : 9 mm

Beispiel II

Dragées mit 5 mg 5,10-Dihydro-5-[[(1-methyl-4-piperidinyl)-amino]carbonyl]-11H-dibenzo[b,e][1,4]diazepin-11-on

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.

Dragéegewicht : 300 mg

## Beispiel III

Ampullen mit 1 mg 5,10-Dihydro-5-[[(1-methyl-4-piperidinyl)-amino]carbonyl]-11H-dibenzo[b,e][1,4]diazepin-11-on-hydrochlorid

Zusammensetzung :
1 Ampulle enthält :

| | |
|---|---|
| Wirkstoff | 1,0 mg |
| Natriumchlorid | 8,0 mg |
| Dest. Wasser | ad 1 ml |

Herstellungsverfahren :

Die Wirksubstanz und Natriumchlorid werden in dest. Wasser gelöst und anschließend auf das gegebene Volumen aufgefüllt. Die Lösung wird sterilfiltriert und in 1 ml-Ampullen abgefüllt.

Sterilisation : 20 Minuten bei 120 °C.

## Beispiel IV

Suppositorien mit 5 mg 5,10-Dihydro-5-[[(1-methyl-4-piperidinyl)amino]carbonyl]-11H-dibenzo[b,e][1,4]diazepin-11-on

Zusammensetzung :
1 Zäpfchen enthält :

| | |
|---|---|
| Wirkstoff | 5,0 mg |
| Zäpfchenmasse (z. B. Witepsol W 45 R) | 1 695,0 mg |
| | 1 700,0 mg |

Herstellungsverfahren :

Die feinpulverisierte Wirksubstanz wird in der geschmolzenen und auf 40 °C abgekühlten Zäpfchenmasse suspendiert. Man gießt die Masse bei 37 °C in leicht vorgekühlte Zäpfchenformen aus.

Zäpfchengewicht : 1,7 g

## Beispiel V

Tropfen mit 5,10-Dihydro-5-[[(1-methyl-4-piperidinyl)amino]-carbonyl]-11H-dibenzo[b,e][1,4]diazepin-11-on enthaltend 0,5 g Wirkstoff/100 ml Lösung

Zusammensetzung :
100 ml Tropflösung enthalten :

| | |
|---|---|
| p-Hydroxybenzoesäuremethylester | 0,035 g |
| p-Hydroxybenzoesäurepropylester | 0,015 g |
| Anisöl | 0,05 g |
| Menthol | 0,06 g |
| Ethanol rein | 10,0 g |
| Wirkstoff | 0,5 g |
| Natriumcyclamat | 1,0 g |
| Glycerin | 15,0 g |
| Dest. Wasser ad | 100,0 ml |

Herstellungsverfahren :

Die Wirksubstanz und Natriumcyclamat werden in ca. 70 ml Wasser gelöst und Glycerin zugefügt. Man löst die p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

**Patentansprüche** (für die Vertragsstaaten : BE, CH, DE, FR, GB, IT, LI, LU, NL, SE)

1. Substituierte Dibenzodiazepinone der allgemeinen Formel I

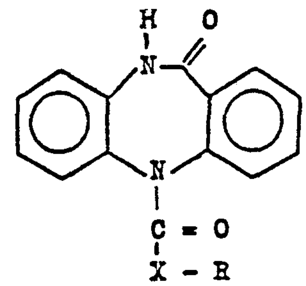

(I)

in der

X ein Sauerstoffatom, eine —NH— oder —$NCH_3$-Gruppe und

R eine gegebenenfalls durch eine weitere Methylgruppe substituierte 1-Methyl-4-piperidinyl-, 4-Methyl-1-piperazinyl-, endo-8-Methyl-8-azabicyclo[3,2,1]oct-3-yl oder exo-8-Methyl-8-azabicyclo[3,2,1]oct-3-yl-gruppe bedeuten, gegebenenfalls ihre diastereomeren und enantiomeren Formen und ihre physiologisch verträglichen Säureadditionssalze mit anorganischen oder organischen Säuren.

2. Substituierte Dibenzodiazepinone der allgemeinen Formel I gemäß Anspruch 1, dadurch gekennzeichnet, daß

X ein Sauerstoffatom oder eine —NH-Gruppe und

R eine 1-Methyl-4-piperidinyl- oder 4-Methyl-1-piperazinyl-gruppe bedeuten,

und ihre physiologisch verträglichen Säureadditionssalze mit anorganischen oder organischen Säuren.

3. Arzneimittel, enthaltend eine oder mehrere Verbindungen der allgemeinen Formel I gemäß den Ansprüchen 1 und 2 neben den üblichen Träger- und/oder Hilfsstoffen.

4. Verfahren zur Herstellung substituierter Dibenzodiazepinone der allgemeinen Formel I

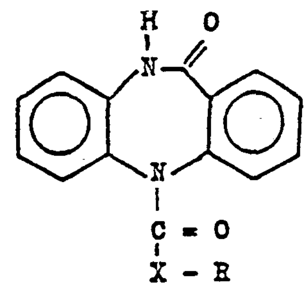

(I)

in der

X ein Sauerstoffatom, eine —NH— oder $NCH_3$-Gruppe und

R eine gegebenenfalls durch eine weitere Methylgruppe substituierte 1-Methyl-4-piperidinyl-, 4-Methyl-1-piperazinyl-, endo-8-Methyl-8-azabicyclo[3,2,1]oct-3-yl- oder exo-8-Methyl-8-azabicyclo[3,2,1]oct-3-yl-Gruppe bedeuten, und von ihren Säureadditionssalzen mit anorganischen oder organischen Säuren, dadurch gekennzeichnet, daß

a) ein Dibenzodiazepinon der allgemeinen Formel Ia

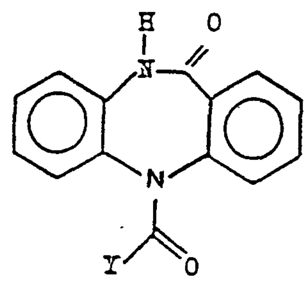

(Ia)

in der Y ein Halogenatom oder die Gruppe $OR_1$ bedeutet, worin $R_1$ einen gegebenenfalls halogensubstituierten Alkylrest mit 1 bis 5 Kohlenstoffatomen, einen gegebenenfalls durch Halogen oder Nitro substituierten Phenylrest oder einen Aralkylrest mit 7 bis 15 Kohlenstoffatomen darstellt, mit einer Verbindung der allgemeinen Formeln

$$R—X—R \qquad (II)$$

oder

$$M—X—R \qquad (IIa)$$

in denen R und X wie oben angegeben definiert sind und M ein Alkalimetallatom oder 1 Äquivalent eines Erdalkalimetallatoms bedeutet, gegebenenfalls in Gegenwart eines Lösungsmittels bei Temperaturen zwischen 0 °C und dem Siedepunkt des Reaktionsgemisches umgesetzt wird oder

b) 5,10-Dihydro-11H-dibenzo[b,e][1,4]diazepin-11-on mit einem Chlorkohlensäurederivat der allgemeinen Formel IV

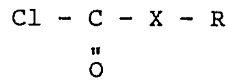

(IV)

bzw. mit einem Isocyanat der allgemeinen Formel IVa

$$O = C = N—R \qquad \text{(IVa)}$$

in denen R und X wie oben angegeben definiert sind, in organischen Lösungsmitteln, gegebenenfalls in Gegenwart von tertiären organischen Basen, bei Temperaturen zwischen 30 °C und dem Siedepunkt des Reaktionsgemisches umgesetzt werden, oder

c) 5,10-Dihydro-11H-dibenzo[b,e][1,4]-diazepin-11-on in einem organischen Lösungsmittel zuerst mit mindestens 2 Äquivalenten eines Lithiumalkyls oder Lithiumaryls bei Temperaturen zwischen – 60 und 0 °C und anschließend mit einem Ester der allgemeinen Formel V

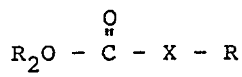

(V)

in der X und R wie oben definiert sind und $R_2$ einen Alkylrest mit 1 bis 10 Kohlenstoffatomen oder einen Aralkylrest mit 7 bis 13 Kohlenstoffatomen darstellt, unter Isolierung des Endproduktes umgesetzt wird, und gegebenenfalls so erhaltene Verbindungen der allgemeinen Formel I anschließend in ihre Salze mit anorganischen oder organischen Säuren übergeführt werden.

5. Verfahren gemäß Anspruch 4a, dadurch gekennzeichnet, daß die Dibenzodiazepinone der allgemeinen Formel Ia mit den Verbindungen der allgemeinen Formel II in Gegenwart von Wasser, Toluol, von Alkoholen, von aprotischen, polaren Lösungsmitteln oder in Gemischen solcher Lösungsmitteln bei Temperaturen von 40 bis 100 °C und in Gegenwart anorganischer oder organischer Basen oder in Gegenwart eines Überschusses der Verbindungen der allgemeinen Formel II zur Reaktion gebracht werden.

6. Verfahren gemäß Anspruch 4b, dadurch gekennzeichnet, daß die Umsetzung in aromatischen Kohlenwasserstoffen, in Acetonitril, Dimethylformamid oder in Gemischen dieser Lösungsmittel bei Temperaturen zwischen 30 und 100 °C erfolgt.

7. Verfahren gemäß Anspruch 4c, dadurch gekennzeichnet, daß die Reaktion bei – 10 °C durchgeführt wird.

8. Verfahren gemäß Anspruch 4c, dadurch gekennzeichnet, daß als Lithiumalkyl oder -aryl n-Butyllithium, n-Butyllithium in Gegenwart von Tetramethylendiamin, tertiär-Butyllithium, Lithiumdiisopropylamid, Lithiumdicyclohexylamid oder Lithiumphenyl und als Lösungsmittel Ether, wie Diethylether, Tetrahydrofuran, aliphatische Kohlenwasserstoffe, wie Hexan, Gemische dieser Lösungsmittel, als Colsolvens Hexamethylphosphoramid verwendet wird.

9. Die Verwendung von Verbindungen der allgemeinen Formel I gemäß Anspruch 1 und ihrer physiologisch verträglichen Salze zur Herstellung von Arzneimitteln.

**Patentansprüche** (für den Vertragsstaat AT)

1. Verfahren zur Herstellung substituierter Dibenzodiazepinone der allgemeinen Formel I

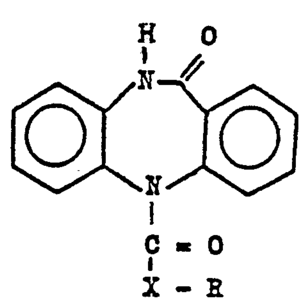

(I)

in der

X ein Sauerstoffatom, eine —NH— oder $NCH_3$-Gruppe und

R eine gegebenenfalls durch eine weitere Methylgruppe substituierte 1-Methyl-4-piperidinyl-, 4-Methyl-1-piperazinyl-, endo-8-Methyl-8-azabicyclo[3,2,1]oct-3-yl- oder exo-8-Methyl-8-azabicyclo[3,2,1]oct-3-yl-Gruppe bedeuten, und von ihren Säureadditionssalzen mit anorganischen oder organischen Säuren, dadurch gekennzeichnet, daß

a) ein Dibenzodiazepinon der allgemeinen Formel Ia

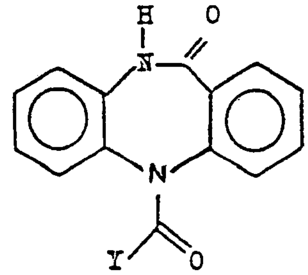

(Ia)

in der Y ein Halogenatom oder die Gruppe $OR_1$ bedeutet, worin $R_1$ einen gegebenenfalls halogensubstituierten Alkylrest mit 1 bis 5 Kohlenstoffatomen, einen gegebenenfalls durch Halogen oder Nitro substituierten Phenylrest oder einen Aralkylrest mit 7 bis 15 Kohlenstoffatomen darstellt, mit einer Verbindung der allgemeinen Formeln

$$H—X—R \qquad (II)$$

oder

$$M—X—R \qquad (IIa)$$

in denen R und X wie oben angegeben definiert sind und M ein Alkalimetallatom oder 1 Äquivalent eines Erdalkalimetallatoms bedeutet, gegebenenfalls in Gegenwart eines Lösungsmittels bei Temperaturen zwischen 0 °C und dem Siedepunkt des Reaktionsgemisches umgesetzt wird oder

b) 5,10-Dihydro-11H-dibenzo[b,e][1,4]diazepin-11-on mit einem Chlorkohlensäurederivat der allgemeinen Formel IV

$$Cl - \underset{\underset{O}{\|}}{C} - X - R \qquad (IV)$$

bzw. mit einem Isocyanat der allgemeinen Formel IVa

$$O=C=N—R \qquad (IVa)$$

in denen R und X wie oben angegeben definiert sind, in organischen Lösungsmitteln, gegebenenfalls in Gegenwart von tertiären organischen Basen, bei Temperaturen zwischen 30 °C und dem Siedepunkt des Reaktionsgemisches umgesetzt werden, oder

c) 5,10-Dihydro-11H-dibenzo[b,e][1,4]diazepin-11-on in einem organischen Lösungsmittel zuerst mit mindestens 2 Äquivalenten eines Lithiumalkyls oder Lithiumaryls bei Temperaturen zwischen − 60 und 0 °C und anschließend mit einem Ester der allgemeinen Formel V

$$R_2O - \overset{\overset{O}{\|}}{C} - X - R \qquad (V)$$

in der X und R wie oben definiert sind und $R_2$ einen Alkylrest mit 1 bis 10 Kohlenstoffatomen oder einen Aralkylrest mit 7 bis 13 Kohlenstoffatomen darstellt, unter Isolierung des Endproduktes umgesetzt wird, und gegebenenfalls so erhaltene Verbindungen der allgemeinen Formel I anschließend in ihre Salze mit anorganischen oder organischen Säuren übergeführt werden.

2. Verfahren zur Herstellung, substituierter Dibenzodiazepinone der allgemeinen Formel I

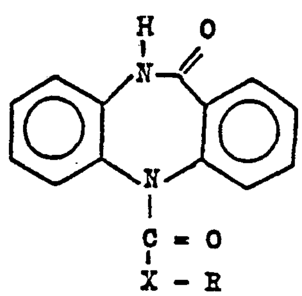

(I)

in der X und R wie im Anspruch 1 definiert sind, und von ihren Säureadditionssalzen mit anorganischen oder organischen Säuren, dadurch gekennzeichnet, daß

a) ein Dibenzodiazepinon der allgemeinen Formel Ia

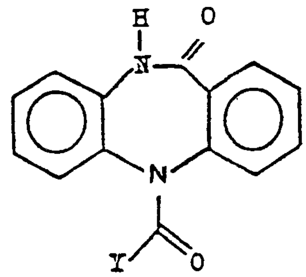

(Ia)

in der Y ein Halogenatom oder die Gruppe $OR_1$ bedeutet, worin $R_1$ einen gegebenenfalls halogensubstituierten Alkylrest mit 1 bis 5 Kohlenstoffatomen, einen gegebenenfalls durch Halogen oder Nitro substituierten Phenylrest oder einen Aralkylrest mit 7 bis 15 Kohlenstoffatomen darstellt, mit einer Verbindung der allgemeinen Formeln

$$H—X—R \qquad (II)$$

oder

$$M—X—R \qquad (IIa)$$

in denen R und X wie oben angegeben definiert sind und M ein Alkalimetallatom oder 1 Äquivalent eines Erdalkalimetallatoms bedeutet, gegebenenfalls in Gegenwart eines Lösungsmittels bei Temperaturen zwischen 0 °C und dem Siedepunkt des Reaktionsgemisches umgesetzt wird oder

b) 5,10-Dihydro-11H-dibenzo[b,e][1,4]diazepin-11-on mit einem Chlorkohlensäurederivat der allgemeinen Formel IV

$$Cl - \underset{\overset{\|}{O}}{C} - X - R \qquad (IV)$$

bzw. mit einem Isocyanat der allgemeinen Formel IVa

$$O = C = N—R \qquad (IVa)$$

in denen R und X wie oben angegeben definiert sind, in organischen Lösungsmitteln, gegebenenfalls in Gegenwart von tertiären organischen Basen, bei Temperaturen zwischen 30 °C und dem Siedepunkt des Reaktionsgemisches umgesetzt werden, und gegebenenfalls so erhaltene Verbindungen der allgemeinen Formel I anschließend in ihre Salze mit anorganischen oder organischen Säuren übergeführt werden.

3. Verfahren gemäß Anspruch 1a, dadurch gekennzeichnet, daß die Dibenzodiazepinone der allgemeinen Formel Ia mit den Verbindungen der allgemeinen Formel II in Gegenwart von Wasser, Toluol, von Alkoholen, von aprotischen polaren Lösungsmitteln oder in Gemischen solcher Lösungsmitteln bei Temperaturen von 40 bis 100 °C und in Gegenwart anorganischer oder organischer Basen oder in Gegenwart eines Überschusses der Verbindungen der allgemeinen Formel II zur Reaktion gebracht werden.

4. Verfahren gemäß Anspruch 1b, dadurch gekennzeichnet, daß die Umsetzung in aromatischen Kohlenwasserstoffen, in Acetonitril, Dimethylformamid oder in Gemischen dieser Lösungsmittel bei Temperaturen zwischen 30 und 100 °C erfolgt.

5. Verfahren gemäß Anspruch 1c, dadurch gekennzeichnet, daß die Reaktion bei − 10 °C durchgeführt wird.

6. Verfahren gemäß Anspruch 1c, dadurch gekennzeichnet, daß als Lithiumalkyl oder -aryl n-Butyllithium, n-Butyllithium in Gegenwart von Tetramethylendiamin, tertiär-Butyllithium, Lithiumdiisopropylamid, Lithiumdicyclohexylamid oder Lithiumphenyl und als Lösungsmittel Ether, wie Diethylether, Tetrahydrofuran, aliphatische Kohlenwasserstoffe, wie Hexan, Gemische dieser Lösungsmittel, als Colsolvens Hexamethylphosphoramid verwendet wird.

**Claims** (for the Contracting States : BE, CH, DE, FR, GB, IT, LI, LU, NL, SE)

1. Substituted dibenzodiazepinones of general formula I

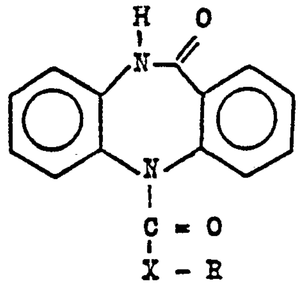

(I)

wherein

X represents an oxygen atom, an —NH— or —$NCH_3$— group and

R represents a 1-methyl-4-piperidinyl, 4-methyl-1-piperazinyl, endo-8-methyl-8-azabicyclo[3,2,1]oct-3-yl or exo-8-methyl-8-azabicyclo [3,2,1] oct-3-yl group, optionally substituted by another methyl group, and optionally the diastereomeric and enantiomeric forms thereof and the physiologically acceptable acid addition salts thereof with inorganic or organic acids.

2. Substituted dibenzodiazepinones of general I as claimed in claim 1, characterised in that

X represents an oxygen atom or an —NH— group and

R represents a 1-methyl-4-piperidinyl or 4-methyl-1-piperazinyl group,

and the physiologically acceptable acid addition salts thereof with inorganic or organic acids.

3. Pharmaceutical compositions containing one or more compounds of general formula I as claimed in claims 1 and 2 together with the usual carriers and/or excipients.

4. Process for preparing substituted dibenzodiazepinones of general formula I

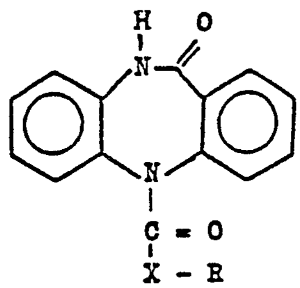

(I)

wherein

X represents an oxygen atom, an —NH— or —$NCH_3$— group and

R represents a 1-methyl-4-piperidinyl, 4-methyl-1-piperazinyl, endo-8-methyl-8-azabicyclo[3,2,1]oct-3-yl or exo-8-methyl-8-azabicyclo[3,2,1]oct-3-yl group, optionally substituted by another methyl group, and of the acid addition salts thereof with inorganic or organic acids, characterised in that

a) a dibenzodiazepinone of general formula Ia

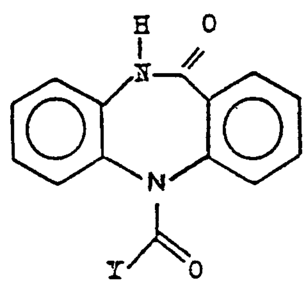

(Ia)

wherein Y represents a halogen atom or the group $OR_1$ wherein $R_1$ represents an optionally halogen-substituted alkyl group with 1 to 5 carbon atoms, a phenyl group optionally substituted by halogen or nitro or an aralkyl group with 7 to 15 carbon atoms, is reacted with a compound of general formula

H—X—R (II)

or

M—X—R (IIa)

wherein R and X are as hereinbefore defined and M represents an alkali metal atom or one equivalent of an alkaline earth metal atom, optionally in the presence of a solvent at temperatures of between 0 °C and the boiling point of the reaction mixture, or

b) 5,10-Dihydro-11H-dibenzo[b,e][1,4]diazepin-11-one is reacted with a chlorocarbonic acid derivative of general formula IV

$$\mathrm{Cl-\underset{\underset{O}{\|}}{C}-X-R} \qquad (IV)$$

or with an isocyanate of general formula IVa

$$O=C=N—R \qquad (IVa)$$

wherein R and X are as hereinbefore defined, in organic solvents, optionally in the presence of tertiary organic bases, at temperatures of between 30 °C and the boiling point of the reaction mixture, or

c) 5,10-Dihydro-11H-dibenzo[b,e][1,4]diazepin-11-one is reacted in an organic solvent, first with at least two equivalents of a lithium alkyl or lithium aryl at temperatures of between − 60 and 0 °C and subsequently with an ester of general formula V

$$\mathrm{R_2O-\overset{\overset{O}{\|}}{C}-X-R} \qquad (V)$$

wherein X and R are as hereinbefore defined and $R_2$ represents an alkyl group with 1 to 10 carbon atoms or an aralkyl group with 7 to 13 carbon atoms, the end product being isolated, and if desired compounds of general formula I thus obtained are subsequently converted into the salts thereof with inorganic or organic acids.

5. Process as claimed in claim 4a, characterised in that the dibenzodiazepinones of general formula Ia are reacted with the compounds of general formula II in the presence of water, toluene, alcohols, aprotic polar solvents or in mixtures of such solvents at temperatures of from 40 to 100 °C and in the presence of inorganic or organic bases or in the presence of an excess of the compounds of general formula II.

6. Process as claimed in claim 4b, characterised in that the reaction is carried out in aromatic hydrocarbons, in acetonitrile, dimethylformamide or in mixtures of these solvents, at temperatures of between 30 and 100 °C.

7. Process as claimed in claim 4c, characterised in that the reaction is carried out at − 10 °C.

8. Process as claimed in claim 4c, characterised in that the lithium alkyl or lithium aryl used is n-butyllithium, n-butyllithium in the presence of tetramethylene diamine, tert.butyl-lithium, lithium diisopropylamide, lithium dicyclohexylamide or lithium phenyl and the solvent used is an ether such as diethyl ether, tetrahydrofuran, aliphatic hydrocarbons such as hexane, or mixtures of these solvents, whilst hexamethylphosphoramide is used as a cosolvent.

9. The use of compounds of general formula I as claimed in claim 1 and the physiologically acceptable salts thereof for the preparation of pharmaceutical compositions.

**Claims** (for the Contracting State AT)

1. Process for preparing substituted dibenzodiazepinones of general formula I

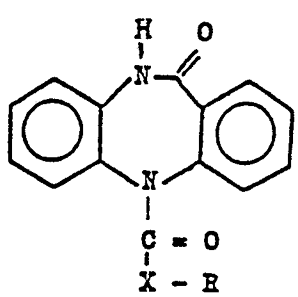

(I)

wherein

X represents an oxygen atom, an —NH— or —$NCH_3$— group and

R represents a 1-methyl-4-piperidinyl, 4-methyl-1-piperazinyl, endo-8-methyl-8-azabicyclo[3,2,1]oct-3-yl or exo-8-methyl-8-azabicyclo[3,2,1]oct-3-yl group, optionally substituted by another methyl group, and of the acid addition salts thereof with inorganic or organic acids, characterised in that

a) a dibenzodiazepinone of general formula Ia

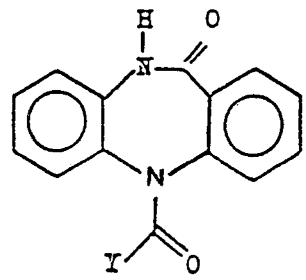

(Ia)

wherein Y represents a halogen atom or the group $OR_1$ wherein $R_1$ represents an optionally halogen-substituted alkyl group with 1 to 5 carbon atoms, a phenyl group optionally substituted by halogen or nitro or an aralkyl group with 7 to 15 carbon atoms, is reacted with a compound of general formula

$$H—X—R \qquad (II)$$

or

$$M—X—R \qquad (IIa)$$

wherein R and X are as hereinbefore defined and M represents an alkali metal atom or one equivalent of an alkaline earth metal atom, optionally in the presence of a solvent at temperatures of between 0 °C and the boiling point of the reaction mixture, or

b) 5,10-Dihydro-11H-dibenzo[b,e][1,4]diazepin-11-one is reacted with a chlorocarbonic acid derivative of general formula IV

```
Cl - C - X - R
     "
     O
```

(IV)

or with an isocyanate of general formula IVa

$$O = C = N—R \qquad (IVa)$$

wherein R and X are as hereinbefore defined, in organic solvents, optionally in the presence of tertiary organic bases, at temperatures of between 30 °C and the boiling point of the reaction mixture, or

c) 5,10-Dihydro-11H-dibenzo[b,e][1,4]diazepin-11-one is reacted in an organic solvent, first with at least two equivalents of a lithium alkyl or lithium aryl at temperatures of between − 60 and 0 °C and subsequently with an ester of general formula V

```
        O
        "
R2O - C - X - R
```

(V)

wherein X and R are as hereinbefore defined and $R_2$ represents an alkyl group with 1 to 10 carbon atoms or an aralkyl group with 7 to 13 carbon atoms, the end product being isolated, and if desired compounds of general formula I thus obtained are subsequently converted into the salts thereof with inorganic or organic acids.

2. Process for preparing substituted dibenzodiazepinones of general formula I

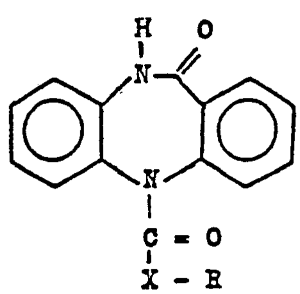

(I)

wherein X and R are defined as in claim 1, and the acid addition salts thereof with inorganic or organic acids, characterised in that

a) a dibenzodiazepinone of general formula Ia

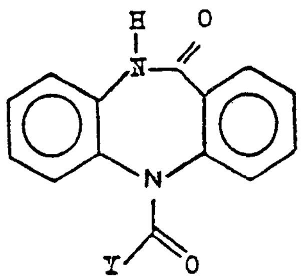

(Ia)

wherein Y represents a halogen atom or the group $OR_1$ wherein $R_1$ represents an optionally halogen-substituted alkyl group with 1 to 5 carbon atoms, a phenyl group optionally substituted by halogen or nitro or an aralkyl group with 7 to 15 carbon atoms, is reacted with a compound of general formula

$$H—X—R \qquad (II)$$

or

$$M—X—R \qquad (IIa)$$

wherein R and X are as hereinbefore defined and M represents an alkali metal atom or one equivalent of an alkaline earth metal atom, optionally in the presence of a solvent at temperatures of between 0 °C and the boiling point of the reaction mixture, or

b) 5,10-Dihydro-11H-dibenzo[b,e][1,4]diazepin-11-one is reacted with a chlorocarbonic acid derivative of general formula IV

$$Cl-\underset{\underset{O}{\|}}{C}-X-R \qquad (IV)$$

or with an isocyanate of general formula IVa

$$O=C=C=N—R \qquad (IVa)$$

wherein R and X are as hereinbefore defined, in organic solvents, optionally in the presence of tertiary organic bases, at temperatures of between 30 °C and the boiling point of the reaction mixture, and if desired compounds of general formula I thus obtained are subsequently converted into the salts thereof with inorganic or organic acids.

3. Process as claimed in claim 1a, characterised in that the dibenzodiazepinones of general formula Ia are reacted with the compounds of general formula II in the presence of water, toluene, alcohols, aprotic polar solvents or in mixtures of such solvents at temperatures of from 40 to 100 °C and in the presence of inorganic or organic bases or in the presence of an excess of the compounds of general formula II.

4. Process as claimed in claim 1b, characterised in that the reaction is carried out in aromatic hydrocarbons, in acetonitrile, dimethylformamide or in mixtures of these solvents, at temperatures of between 30 and 100 °C.

5. Process as claimed in claim 1c, characterised in that the reaction is carried out at − 10 °C.

6. Process as claimed in claim 1c, characterised in that the lithium alkyl or lithium aryl used is n-butyllithium, n-butyllithium in the presence of tetramethylene diamine, tert.butyl-lithium, lithium diisopropylamide, lithium dicyclohexylamide or lithium phenyl and the solvent used in an ether such as diethyl ether, tetrahydrofuran, aliphatic hydrocarbons such as hexane, or mixtures of these solvents, whilst hexamethylphosphoramide is used as a cosolvent.

**Revendications** (pour les Etats contractants : BE, CH, DE, FR, GB, IT, LI, LU, NL, SE)

1. Dibenzodiazépinones substituées de formule générale I

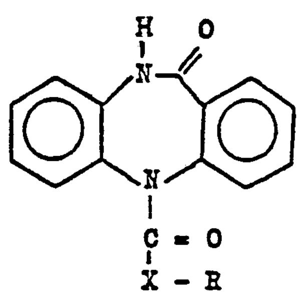

(I)

dans laquelle

X représente un atome d'oxygène, un groupe —NH— ou —$NCH_3$— et

R représente un groupe 1-méthyl-4-pipéridinyle, 4-méthyl-1-pipérazinyle, endo-8-méthyl-8-azabicyclo[3,2,1]-oct-3-yle ou exo-8-méthyl-8-azabicyclo[3,2,1]oct-3-yle éventuellement substitué par un autre groupe méthyle, éventuellement leurs formes diastéréoisomères et énantionères et leurs sels d'addition d'acides physiologiquement supportables avec des acides minéraux ou organiques.

2. Dibenzodiazépinones substituées de formule générale I selon la revendication 1, caractérisées en ce que

X représente un atome d'oxygène ou un groupe —NH— et

R représente un groupe 1-méthyl-4-pipéridinyle ou 4-méthyl-1-pipérazinyle,

et leurs sels d'addition d'acides physiologiquement supportables avec des acides minéraux ou organiques.

3. Médicaments contenant un ou plusieurs composés de formule générale I selon les revendications 1 et 2, conjointement aux excipients et/ou adjuvants usuels.

4. Procédé pour la préparation de dibenzodiazépinones substituées de formule générale I

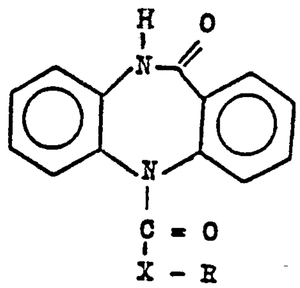

(I)

dans laquelle

X représente un atome d'oxygène, un groupe —NH— ou —$NCH_3$— et

R représente un groupe 1-méthyl-4-pipéridinyle, 4-méthyl-1-pipérazinyle, endo-8-méthyl-8-azabicyclo[3,2,1]oct-3-yle ou exo-8-méthyl-8-azabicyclo[3,2,1]oct-3-yle, éventuellement substitué par un autre groupe méthyle, et de leurs sels d'addition d'acides avec des acides minéraux ou organiques, caractérisé en ce que :

a) on fait réagir une dibenzodiazépinone de formule générale Ia

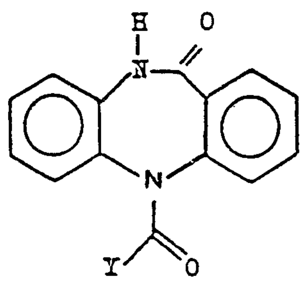

(Ia)

dans laquelle Y représente un atome d'halogène ou le groupe $OR_1$, dans lequel $R_1$ représente un radical alcoyle avec 1 à 5 atomes de carbone, éventuellement halogénosubstitué, ou représente un radical phényle éventuellement substitué par halogène ou nitro ou représente un radical aralcoyle avec 7 à 15 atomes de carbone, avec un composé de formules générales

H—X—R (II)

ou

M—X—R (IIa)

dans lesquelles R et X sont définis comme indiqué plus haut et M représente un atome de métal alcalin ou 1 équivalent d'un atome de métal alcalino-terreux, éventuellement en présence d'un solvant à des températures entre 0 °C et le point d'ébullition du mélange réactionnel ou

b) on fait réagir la 5,10-dihydro-11H-dibenzo[b,e][1,4]diazépine-11-one avec un dérivé d'acide chlorocarboxylique de formule générale IV

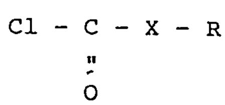

(IV)

ou avec un isocyanate de formule générale IVa

$$O = C = N—R \qquad (IVa)$$

dans lesquelles R et X sont définis comme indiqué plus haut, dans des solvants organiques, éventuellement en présence de bases organiques tertiaires, à des températures entre 30 °C et le point d'ébullition du mélange réactionnel, ou

c) on fait réagir la 5,10-dihydro-11H-dibenzo[b,e][1,4]-diazépine-11-one dans un solvant organique, d'abord avec au moins 2 équivalents d'un lithiumalcoyle ou lithiumaryle à des températures entre – 60 et 0 °C et ensuite avec un ester de formule générale V

$$R_2O - \overset{\overset{O}{\|}}{C} - X - R \qquad (V)$$

dans laquelle X et R sont définis comme plus haut et $R_2$ représente un radical alcoyle avec 1 à 10 atomes de carbone ou un radical aralcoyle avec 7 à 13 atomes de carbone, en isolant le produit final, et éventuellement on transforme ensuite des composés ainsi obtenus de formule générale I en leurs sels avec des acides minéraux ou organiques.

5. Procédé selon la revendication 4a, caractérisé en ce que les dibenzodiazépinones de formule générale Ia sont mises à réagir avec les composés de formule générale II en présence d'eau, de toluène, d'alcools, de solvants polaires aprotiques ou dans des mélanges de tels solvants à des températures de 40 à 100 °C et en présence de bases minérales ou organiques ou en présence d'un excès des composés de formule générale II.

6. Procédé selon la revendication 4b, caractérisé en ce que la réaction a lieu dans des hydrocarbures aromatiques, dans l'acétonitrile, le diméthylformamide ou dans des mélanges de ces solvants à des températures entre 30 et 100 °C.

7. Procédé selon la revendication 4c, caractérisé en ce que la réaction est effectuée à – 10 °C.

8. Procédé selon la revendication 4c, caractérisé en ce que l'on utilise en tant que lithium-alcoyle ou -aryle, le n-butyllithium, le n-butyllithium en présence de tétraméthylènediamine, le tertio-butyllithium, le lithiumdiisopropylamide, le lithiumdicyclohexylamide ou le lithiumphényle et en tant que solvant, des éthers comme l'éther éthylique, le tétrahydrofuranne, des hydrocarbures aliphatiques comme l'hexane, des mélanges de ces solvants, comme cosolvant, l'hexaméthylphosphotriamide.

9. Utilisation de composés de formule générale I selon la revendication 1 et de leurs sels physiologiquement supportables pour la préparation de médicaments.

**Revendications** (pour l'Etat contractant AT)

1. Procédé pour la préparation de dibenzodiazépinones substituées de formule générale I

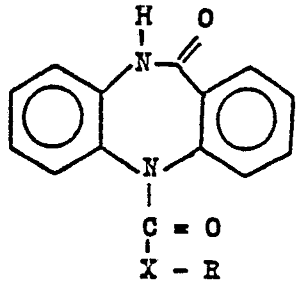

(I)

dans laquelle

X représente un atome d'oxygène, un groupe —NH— ou —$NCH_3$— et

R représente un groupe 1-méthyl-4-pipéridinyle, 4-méthyl-1-pipérazinyle, endo-8-méthyl-8-azabicyclo[3,2,1]oct-3-yle ou exo-8-méthyl-8-azabicyclo[3,2,1]oct-3-yle, éventuellement substitué par un autre groupe méthyle, et de leurs sels d'addition d'acides avec des acides minéraux ou organiques, caractérisé en ce que :

a) on fait réagir une dibenzodiazépinone de formule générale Ia

(Siehe Schema Seite 23 f.)

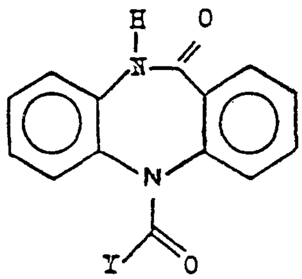

(Ia)

dans laquelle Y représente un atome d'halogène ou le groupe $OR_1$, dans lequel $R_1$ représente un radical alcoyle avec 1 à 5 atomes de carbone, éventuellement halogénosubstitué, ou représente un radical phényle éventuellement substitué par halogène ou nitro ou représente un radical aralcoyle avec 7 à 15 atomes de carbone, avec un composé de formules générales

$$H—X—R \qquad (II)$$

ou

$$M—X—R \qquad (IIa)$$

dans lesquelles R et X sont définis comme indiqué plus haut et M représente un atome de métal alcalin ou 1 équivalent d'un atome de métal alcalino-terreux, éventuellement en présence d'un solvant à des températures entre 0 °C et le point d'ébullition du mélange réactionnel ou

b) on fait réagir la 5,10-dihydro-11H-dibenzo[b,e][1,4]diazépine-11-one avec un dérivé d'acide chlorocarboxylique de formule générale IV

$$Cl - \underset{\underset{O}{\parallel}}{C} - X - R \qquad (IV)$$

ou avec un isocyanate de formule générale IVa

$$O = C = N—R \qquad (IVa)$$

dans lesquelles R et X sont définis comme indiqué plus haut, dans des solvants organiques, éventuellement en présence de bases organiques tertiaires, à des températures entre 30 °C et le point d'ébullition du mélange réactionnel, ou

c) on fait réagir la 5,10-dihydro-11H-dibenzo[b,e][1,4]-diazépine-11-one dans un solvant organique, d'abord avec au moins 2 équivalents d'un lithiumalcoyle ou lithiumaryle à des températures entre − 60 et 0 °C et ensuite avec un ester de formule générale V

$$R_2O - \overset{\overset{O}{\parallel}}{C} - X - R \qquad (V)$$

dans laquelle X et R sont définis comme plus haut et $R_2$ représente un radical alcoyle avec 1 à 10 atomes de carbone ou un radical aralcoyle avec 7 à 13 atomes de carbone, en isolant le produit final, et éventuellement on transforme ensuite des composés ainsi obtenus de formule générale I en leurs sels avec des acides minéraux ou organiques.

2. Procédé pour la préparation de dibenzodiazépinones substituées de formule générale I

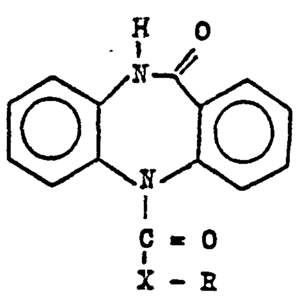

(I)

dans laquelle X et R sont définis comme dans la revendication 1, et de leurs sels d'addition d'acides avec des acides minéraux ou organiques, caractérisé en ce que :

a) on fait réagir une dibenzodiazépinone de formule générale Ia

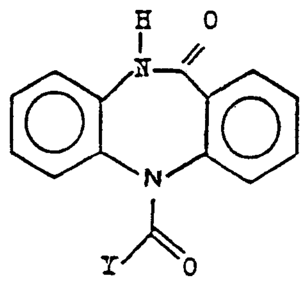

(Ia)

dans laquelle Y représente un atome d'halogène ou le groupe $OR_1$, dans lequel $R_1$ représente un radical alcoyle avec 1 à 5 atomes de carbone, éventuellement halogénosubstitué, ou représente un radical phényle éventuellement substitué par halogène ou nitro ou représente un radical aralcoyle avec 7 à 15 atomes de carbone, avec un composé de formules générales

H—X—R (II)

ou

M—X—R (IIa)

dans lesquelles R et X sont définis comme indiqué plus haut et M représente un atome de métal alcalin ou 1 équivalent d'un atome de métal alcalino-terreux, éventuellement en présence d'un solvant à des températures entre 0 °C et le point d'ébullition du mélange réactionnel ou

b) on fait réagir la 5,10-dihydro-11H-dibenzo[b,e][1,4]-diazépine-11-one avec un dérivé d'acide chlorocarboxylique de formule générale IV

Cl - C - X - R
 ‖
 O (IV)

ou avec un isocyanate de formule générale IVa

O = C = N—R (IVa)

dans lesquelles R et X sont définis comme indiqué plus haut, dans des solvants organiques, éventuellement en présence de bases organiques tertiaires, à des températures entre 30 °C et le point d'ébullition du mélange réactionnel, et éventuellement on transforme ensuite des composés ainsi obtenus de formule générale I en leurs sels avec des acides minéraux ou organiques.

3. Procédé selon la revendication 1a, caractérisé en ce que les dibenzodiazépinones de formule générale Ia sont mises à réagir avec les composés de formule générale II en présence d'eau, de toluène, d'alcools, de solvants polaires aprotiques ou dans des mélanges de tels solvants à des températures de 40 à 100 °C et en présence de bases minérales ou organiques ou en présence d'un excès des composés de formule générale II.

4. Procédé selon la revendication 1b, caractérisé en ce que la réaction a lieu dans des hydrocarbures aromatiques, dans l'acétonitrile, le diméthylformamide ou dans des mélanges de ces solvants à des températures entre 30 et 100 °C.

5. Procédé selon la revendication 1c, caractérisé en ce que la réaction est effectuée à − 10 °C.

6. Procédé selon la revendication 1c, caractérisé en ce que l'on utilise en tant que lithium-alcoyle ou -aryle, le n-butyllithium, le n-butyllithium en présence de tétraméthylènediamine, le tertio-butyllithium, le lithiumdiisopropylamide, le lithiumdicyclohexylamide ou le lithiumphényle et en tant que solvant, des éthers comme l'éther éthylique, le tétrahydrofuranne, des hydrocarbures aliphatiques comme l'hexane, des mélanges de ces solvants, comme cosolvant, l'hexaméthylphosphotriamide.